# EUROPEAN PATENT APPLICATION

(11) **EP 2 993 601 A1**
(43) Date of publication of application: **09.03.2016**
(21) Application number: 15181018.1
(22) Date of filing: 14.08.2015
(51) Int. Cl.: G06F 19/00

(54) **MEDICAL APPARATUS, MEDICAL APPARATUS CONTROL METHOD, AND MEDICAL APPARATUS COOPERATION SYSTEM**

(30) Priority: 04.09.2014 JP 2014180023
(71) Applicant: Nihon Kohden Corporation, Shinjuku-ku Tokyo (JP)
(72) Inventor: Wakabayashi, Tsutomu, Shinjuku-ku, Tokyo (JP); Yoshida, Tatsuo, Shinjuku-ku, Tokyo (JP); Akiyama, Naoto, Shinjuku-ku, Tokyo (JP); Satake, Hiroyuki, Shinjuku-ku, Tokyo (JP)
(74) Representative: Grünecker Patent- und Rechtsanwälte PartG mbB

(57) **Abstract**

A medical apparatus which is communicable with a plurality of CPR assisting devices, the medical apparatus includes: a connection destination determining unit which is configured to determine a connection destination CPR assisting device that is a connection destination, from the plurality of CPR assisting devices based on a predetermined rule; and a communicating unit which is configured to communicate only with the connection destination CPR assisting device.

## Description

### BACKGROUND

The presently disclosed subject matter relates to a medical apparatus, a medical apparatus control method, and a medical apparatus cooperation system.

CPR (Cardio Pulmonary Resuscitation) is a technique which is essential in the field of the emergency medical service, and the life and death of the rescuee (patient) depend on the adequateness of the technique. When CPR is to be performed, the rescuer compresses the sternum which is in the upper side of the chest. Since CPR relates to the life and death of the rescuee, a device which is to be placed between the chest of the rescuee and the hands of the rescuer to assist the chest compression (hereinafter, such a device is referred to as a CPR assisting device) has been developed. In a CPR assisting device, it is detected whether an adequate force is applied at proper time intervals to the sternum or not, and, in accordance with the detection result, appropriate notification (such as "Insufficient compression depth" or "Compression timing is too late") is given to the rescuer.

Such a CPR assisting device has many occasions to be used concurrently with another medical apparatus such as an AED (Automated External Defibrillator) or a cardioverter defibrillator. When a CPR assisting device operates coordinately with a medical apparatus, it is possible to realize an adequate emergency procedure.

For example, JP-T-2013-542814 discloses a technique relating to communication among a plurality of defibrillators, and JP-T-2010-528722 discloses a technique relating to communication between a CPR assisting device and a defibrillator.

In a medical site or a scene of a medical training, it is supposed that many medical apparatuses are concurrently used. When a large-scale disaster (such as a high-rise building fire) occurs, for example, a case where many CPR assisting devices and AEDs (or cardioverter defibrillators) are simultaneously used may be caused. In a training session on CPR or the like, moreover, many CPR assisting devices and AEDs (or cardioverter defibrillators) are simultaneously used.

In the case where, in such a situation, medical apparatuses (for example, a CPR assisting device and an AED) communicate with each other, there is a danger that unintended connection switching may occur. Therefore, there is a possibility that the rescuer who uses the medical apparatuses may be confused.

### SUMMARY

The presently disclosed subject matter may provide a medical apparatus, medical apparatus control method, and medical apparatus cooperation system in which, in the case where medical apparatuses communicate with each other, unintended connection switching can be prevented from occurring.

The medical apparatus which is communicable with a plurality of CPR assisting devices, the medical apparatus may comprise: a connection destination determining unit which is configured to determine a connection destination CPR assisting device that is a connection destination, from the plurality of CPR assisting devices based on a predetermined rule; and a communicating unit which is configured to communicate only with the connection destination CPR assisting device.

### BRIEF DESCRIPTION OF THE DRAWINGS

Fig. 1 is a block diagram of a medical apparatus cooperation system 1 of Embodiment 1.
Fig. 2 is a view showing an example of a connection list which is used by a connection destination determining unit 15 in Embodiment 1.
Fig. 3 is a view showing an example of attribute information which is used by the connection destination determining unit 15 in Embodiment 1.
Fig. 4 is a view showing an example of a communication history list which is used by the connection destination determining unit 15 in Embodiment 1.

### DETAILED DESCRIPTION OF EXEMPLARY EMBODIMENTS

### <Embodiment 1>

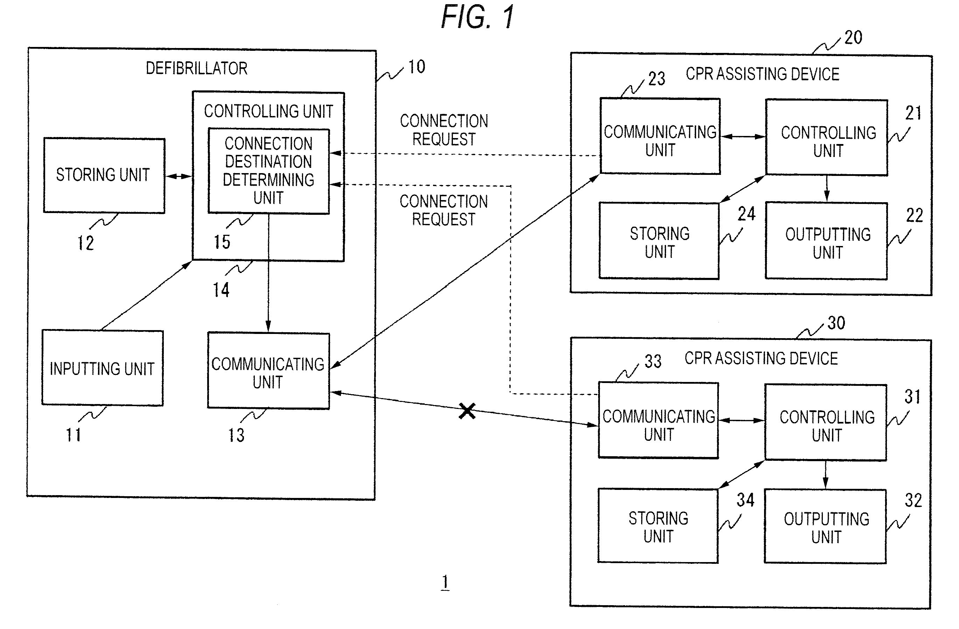

Hereinafter, an embodiment of the presently disclosed subject matter will be described with reference to the drawings. Fig. 1 is a block diagram showing the configuration of a medical apparatus cooperation system 1 of the embodiment. The medical apparatus cooperation system 1 has a defibrillator 10, a CPR assisting device 20, and a CPR assisting device 30. The configuration of the medical apparatus cooperation system 1 shown in Fig. 1 is a mere example, and the defibrillator 10 is an example of a medical apparatus, and may be an AED (Automated External Defibrillator) or the like. Although Fig. 1 shows only the two CPR assisting devices (20, 30), the invention is not limited to this, and three or more CPR assisting devices may exist. In the following description, in the case where the CPR assisting device 20 and the CPR assisting device 30 are not distinct from each other, the devices are not designated by reference numerals, and are referred as "CPR assisting devices."

Firstly, the configuration of the CPR assisting device 20 will be described. The CPR assisting device 20 is a device which is to be placed between the chest (preferably, just above the sternum) of the rescuee and the hands of the rescuer to assist the chest compression. The rescuee is a concept containing an injured or sick human (or alternatively referred to as a patient) and also a mannequin and the like. Namely, the CPR assisting device 20 may be used not only in a situation where the chest compression is actually performed, but also in the training of CPR.

The CPR assisting device 20 includes a controlling unit 21, an outputting unit 22, a communicating unit 23, and a storing unit 24. The CPR assisting device 20 has inside a sensor which is not shown. The sensor detects displacements which are caused when the compression is applied and released, and supplies a signal indicating such a displacement to the controlling unit 21.

The controlling unit 21 performs various processes for controlling the CPR assisting device 20. The controlling unit 21 is realized by a CPU (Central Processing Unit) and various circuits.

Based on the displacement detected by the above-described sensor, the controlling unit 21 detects the compression depth and rate of the chest compression which is applied to the rescuee. The controlling unit 21 reads out ideal compression depth and rate which are stored in the storing unit 24. The controlling unit 21 compares the read-out compression depth with the depth of the currently performed compression, and determines whether the current compression depth is adequate or not. Similarly, the controlling unit 21 compares the read-out compression rate with the rate of the currently performed compression, and determines whether the current compression rate is adequate or not.

If the controlling unit 21 determines that the compression depth or rate of the currently performed compression is not adequate, the controlling unit 21 causes the outputting unit 22 to perform an output in which the abnormal state is informed to the rescuer (user of the CPR assisting device 20). Moreover, the controlling unit 21 transmits the detected compression depth and rate, and various other statuses and the like related to the chest compression to the defibrillator 10 through the communicating unit 23. Only when the defibrillator 10 determines the CPR assisting device 20 as the connection destination as described later, various information can be transmitted to the defibrillator 10.

For example, the techniques of the detection and determination of the compression depth and rate of the chest compression which are performed by the controlling unit 22 may be equivalent to those described in WO2012/073900.

If it is determined that the chest compression is not adequately performed, the outputting unit 22 notifies the user of this situation. For example, the outputting unit 22 may be configured by a speaker, a display device disposed on the surface of the case of the CPR assisting device 20, or the like. In the case where the compression depth is not sufficient, for example, the outputting unit 22 outputs voice guidance instructions of "Press more strongly". In accordance with the determination result, similarly, the outputting unit 22 outputs voice guidance instructions such as "Press more deeply", "Press more slowly", or "Press more quickly". At the same time, the outputting unit 22 may display the status showing the chest compression state on the display device disposed on the surface of the case.

The communicating unit 23 is a communication interface through which various information such as the compression depth and rate of the currently performed compression is transmitted to the defibrillator 10. Before transmitting and receiving data to and from the defibrillator 10, the communicating unit 23 transmits a connection request to the defibrillator 10. The communicating unit 23 receives various information from the defibrillator 10. The communicating unit 23 is an interface which can perform short-range communication such as Bluetooth (registered trademark). The communicating unit 23 transmits the connection request to the defibrillator 10, and, only when the defibrillator 10 determines the CPR assisting device 20 as the connection destination, is allowed to perform data transmission and reception.

The storing unit 24 stores various information (programs which are used by the controlling unit 21, information of the above-described ideal compression depth and rate, and the like). For example, the storing unit 24 is realized by a ROM (Read Only Memory), an HDD (Hard Disk Drive), a USB (Universal Serial Bus) memory, or the like.

The CPR assisting device 30 includes a controlling unit 31, an outputting unit 32, a communicating unit 33, and a storing unit 34. The CPR assisting device 30 has a configuration and function which are approximately identical to those of the CPR assisting device 20. Therefore, the controlling unit 31, the outputting unit 32, the communicating unit 33, and the storing unit 34 correspond to the controlling unit 21, the outputting unit 22, the communicating unit 23, and the storing unit 24, respectively.

Then, the configuration of the defibrillator 10 will be described. The defibrillator 10 includes an inputting unit 11, a storing unit 12, a communicating unit 13, and a controlling unit 14. The controlling unit 14 includes a connection destination determining unit 15. The defibrillator 10 has defibrillation pads and electrodes which are not shown, and applies an electrical shock to the rescuee through the defibrillation pads and the electrodes.

The inputting unit 11 receives various inputs for enabling the user to operate the defibrillator 10. The inputting unit 11 is configured by buttons, knobs, operable display screen (so-called touch display), and the like which are disposed on the surface of the case. For example, the inputting unit 11 receives designation of the charging amount of energy for the defibrillation process which is to be performed on the rescuee.

The storing unit 12 stores various information used for operating the defibrillator 10. The storing unit 12 is realized by a ROM (Read Only Memory), an HDD (Hard Disk Drive), a USB (Universal Serial Bus) memory, or the like.

The controlling unit 14 performs various processes for controlling the defibrillator 10. The controlling unit 14 is realized by a CPU (Central Processing Unit) and various circuits. The controlling unit 14 controls charge/discharge of energy for applying an electrical shock to the rescuee, the sound output, etc.

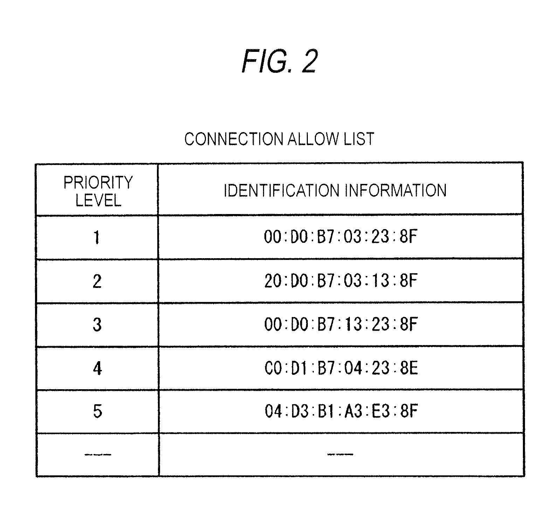

The connection destination determining unit 15 determines the connection destination CPR assisting device which functions as the connection destination, in accordance with a predetermined rule from a plurality of CPR assisting devices (in the example of Fig. 1, the CPR assisting devices 20 and 30) with which the connection destination determining unit 15 can communicate (in other words, which transmit a connection request to the defibrillator 10). Preferably, the connection destination CPR assisting device is a single CPR assisting device. That is, the defibrillator 10 preferably communicates only with a single CPR assisting device. The process of selecting the connection destination by the connection destination determining unit 15 will be described later with reference to Figs. 2 to 4. The connection destination determining unit 15 notifies the communicating unit 13 of identification information (for example, the device address of Bluetooth (registered trademark)) for the determined connection destination CPR assisting device.

As described later, the connection destination determining unit 15 may use one of a plurality of rules for determining the connection destination. Therefore, the user of the defibrillator 10 may designate the rule to be used, through the inputting unit 11.

The communicating unit 13 communicates (performs data transmission and reception) only with the connection destination which is determined by the connection destination determining unit 15. In the example of Fig. 1, the connection destination is the CPR assisting device 20. Therefore, the communicating unit 13 performs data transmission and reception only with respect to the CPR assisting device 20, and does not perform data transmission and reception with respect to the CPR assisting device 30.

Then, methods by which the connection destination determining unit 15 determines the connection destination will be described.

### (Connection destination determination rule 1)

Hereinafter, a first determination rule will be described. The storing unit 12 stores a connection list in which connectable CPR assisting devices are previously defined. The user of the defibrillator 10 sets or changes the connection list through the inputting unit 11. Fig. 2 is a view showing an example of the connection list.

In the connection list, CPR assisting devices which are determined to be connectable with the defibrillator 10 are listed. The connection list manages CPR assisting devices in accordance with identification information for identifying the devices. In the connection list, for example, CPR assisting devices may be managed in accordance with the device address of Bluetooth (registered trademark). As shown in Fig. 2, the connection list may store connectable CPR assisting devices while correlating them with priority levels.

When the connection destination determining unit 15 receives a connection request from a certain CPR assisting device, the unit determines whether the device can be set as a connection destination CPR assisting device or not, depending on whether the identification information of the CPR assisting device is included in the connection list or not. In the case where the connection destination determining unit 15 receives connection requests from a plurality of CPR assisting devices which are included in the connection list, the unit determines the connection destination CPR assisting device based on the priority level. In the example of Fig. 2, it is assumed that the connection destination determining unit 15 simultaneously receives connection requests from a CPR assisting device having identification information of "00:D0:B7:03:23:8F" (priority level = 1), and a CPR assisting device having identification information of "C0:D1:B7:04:23:8E" (priority level = 4). The connection destination determining unit 15 determines the device of "00:D0:B7:03:23:8F" (priority level = 1) as the connection destination CPR assisting device. The identification information is not limited to the device address, but may be of arbitrary format and content.

As described above, the connection destination determining unit 15 is configured so as to allow the defibrillator 10 to be connected only to the preset CPR assisting device. Therefore, the defibrillator 10 is in a state where it can communicate only with the expected connection destination. As a result, it is possible to avoid a situation where the defibrillator 10 is connected to an unintended CPR assisting device, and the user is confused.

In the case where a plurality of CPR assisting devices are set as connection candidates, the connection destination determining unit 15 determines the connection destination based on the priority levels. Therefore, the connection destination determining unit 15 can communicate only with the most appropriate CPR assisting device.

### (Connection destination determination rule 2)

Hereinafter, a second determination rule will be described. The connection destination determining unit 15 determines a CPR assisting device which transmits a connection request at the earliest timing after a predetermined timing (for example, the timing when the defibrillator is powered ON), as the connection destination. In the example of Fig. 1, it is assumed that the CPR assisting device 20 transmits a connection request in advance of the CPR assisting device 30. Therefore, the connection destination determining unit 15 determines the CPR assisting device 20 as the connection destination CPR assisting device. The connection request is, for example, a signal related to a search operation in the case where Bluetooth (registered trademark) is used.

When this rule (a CPR assisting device which transmits a connection request at the earliest timing is set as the connection destination) is used, CPR can be early applied to the rescuee.

### (Connection destination determination rule 3)

Hereinafter, a third determination rule will be described. In the rule, attributes of a plurality of CPR assisting devices which request the connection are analyzed, and the most appropriate CPR assisting device is determined as the connection destination. An example will be described in detail.

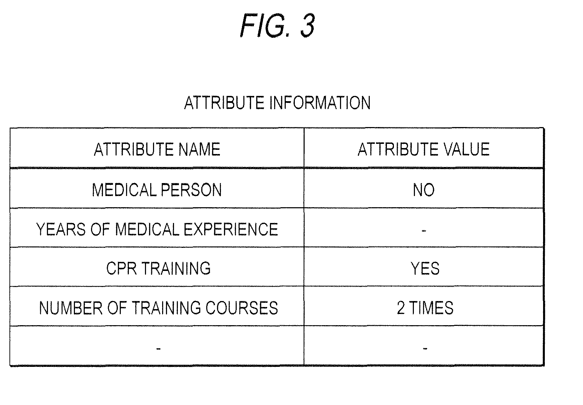

Each CPR assisting device which wishes to communicate with the defibrillator 10 transmits the attribute information of the own device to the defibrillator 10. Fig. 3 shows an example of the attribute information which is transmitted by each CPR assisting device. For example, the attribute information contains "Medical person?", "Years of medical experience", "Has taken CPR training course?", "Number of CPR training courses", and the like. The user of each CPR assisting device previously registers the attribute information.

The connection destination determining unit 15 evaluates the attribute information transmitted from each CPR assisting device. Based on the plural sets of transmitted attribute information, for example, the connection destination determining unit 15 determines a CPR assisting device corresponding to the user who is a medical person, and who has the longest medical experience, as the connection destination CPR assisting device. The connection destination determining unit 15 may evaluate attribute information by using an arbitrary algorithm. For example, the connection destination CPR assisting device may be determined with emphasis on the number of CPR training courses.

As described above, the connection destination determining unit 15 evaluates the attribute information transmitted from the CPR assisting devices, and determines the most appropriate connection destination CPR assisting device. The connection destination determining unit 15 determines the connection destination CPR assisting device based on information (particularly, information of the rescuer) related to each CPR assisting device, and therefore can communicate with the CPR assisting device which is used by the optimum rescuer.

### (Connection destination determination rule 4)

Hereinafter, a fourth determination rule will be described. In the rule, the connection destination CPR assisting device is determined with reference to the history of CPR assisting devices with which the defibrillator 10 has communicated. An example will be described in detail.

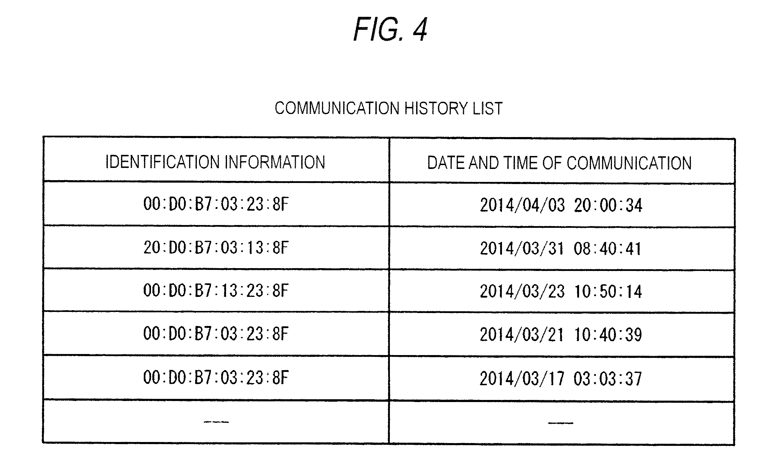

The storing unit 12 stores the identification information of CPR assisting devices with which the defibrillator 10 has communicated, and the dates and times of communications while correlating them with each other. Fig. 4 shows an example of a communication history list stored in the storing unit 12. For example, the identification information for identifying a CPR assisting device may be the device address of Bluetooth (registered trademark).

The connection destination determining unit 15 determines the connection destination CPR assisting device by using the communication history list. For example, the connection destination determining unit 15 may determine, among a plurality of CPR assisting devices which have transmitted a connection request, a CPR assisting device with which the defibrillator 10 has recently communicated, as the connection destination CPR assisting device. Alternatively, the connection destination determining unit 15 may determine, among a plurality of CPR assisting devices which have transmitted a connection request, a CPR assisting device with which the defibrillator 10 has communicated the largest number of times, as the connection destination CPR assisting device.

Since the connection destination determining unit 15 determines the connection destination CPR assisting device with reference to the communication history list, the defibrillator 10 can be connected to a CPR assisting device with which the defibrillator 10 has communicated. In a manner similar to that in the previous used state, therefore, the user can cooperatively use the defibrillator 10 and the CPR assisting device.

### (Connection destination determination rule 5)

Hereinafter, a fifth determination rule will be described. In the rule, the connection destination CPR assisting device is determined by using information of the connection destination which is explicitly designated by the user.

The user of the defibrillator 10 designates a CPR assisting device which is to be communicated with the defibrillator 10, through the inputting unit 11. For example, the user of the defibrillator 10 inputs the identification information of the CPR assisting device which is to be communicated with the defibrillator 10. Alternatively, the defibrillator 10 may display a list of CPR assisting devices which have transmitted a connection request, on the display device or the like disposed on the surface of the case of the defibrillator 10, and the user may designate one of them. The connection destination determining unit 15 determines the CPR assisting device which is designated by the user, as the connection destination CPR assisting device.

As described above, the user can explicitly designate the connection destination CPR assisting device, and therefore the user can perform a rescue procedure on the rescuee by using a pair of the defibrillator 10 and a desired CPR assisting device.

Although, in the above, the five rules for determining the connection destination by the connection destination determining unit 15 have been described, the invention is not limited to the above described determination rules. The connection destination determining unit 15 may use another rule as far as a CPR assisting device to function as the connection destination is determined in accordance with the predetermined rule.

The timing of the process of determining the connection destination CPR assisting device by the connection destination determining unit 15 may be considered as follows. In the case where the connection destination CPR assisting device is once determined, for example, the connection destination determining unit 15 does not change the connection destination CPR assisting device (dose not again perform the determining process) unless a forced reset process is performed (for example, the defibrillator 10 is powered OFF). Here, the process of determining the connection destination CPR assisting device is performed only one time after a predetermined time period has elapsed from when the defibrillator 10 began to operate. Therefore, CPR can be performed without changing the communication destination after the connection destination CPR assisting device is once determined.

Each time when a new CPR assisting device transmits a connection request, the connection destination determining unit 15 may perform the process of determining the connection destination CPR assisting device. Alternatively, the connection destination determining unit 15 may perform the process of determining the connection destination CPR assisting device at regular time intervals. Although there is a possibility that the connection destination is changed during performance of CPR, therefore, it is possible to realize communication with the optimum connection destination. In this case, only when the optimum CPR assisting device newly appears, the connection switching occurs. Therefore, this does not cause unwanted connection switching. In the case where a connection switching occurs, the connection destination determining unit 15 preferably informs the user of the change of the connection destination, through the speaker (not shown) of the defibrillator 10.

Preferably, the user may explicitly designate the timing when the connection destination determining unit 15 determines the connection destination CPR assisting device, through the inputting unit 11 of the defibrillator 10.

Then, effects of the medical apparatus cooperation system of the embodiment will be described. The defibrillator 10 (medical apparatus) has the configuration where it communicates only with the connection destination CPR assisting device which is determined in accordance with the predetermined rule. Therefore, the medical apparatus does not communicate with apparatuses or devices other than the connection destination CPR assisting device. Unintended connection switching can be prevented from occurring, and hence the rescuer can perform CPR without being confused.

Although the invention conducted by the inventor has been specifically described based on the embodiment, the invention is not limited to the above-described embodiment, and it is a matter of course that various changes can be made without departing from the spirit of the invention.

Although, in the above, the operation in which the defibrillator 10 selects the connection destination from the plurality of CPR assisting devices has been described, the configuration (internal configuration of the defibrillator 10 shown in Fig. 1) and algorithm that have been described above may be applied to the reverse case (i.e., the case where the CPR assisting device 20 selects the connection destination from a plurality of defibrillators 10). That is, "configuration which determines an opposing medical apparatus (connection destination opposing medical apparatus) that is to be a connection destination, from a plurality of opposing medical apparatuses based on a predetermined rule (for example, Figs. 2 to 4 above), and which communicates only with the determined connection destination (connection destination opposing medical apparatus)" can be applied to an arbitrary medical apparatus.

According to the presently disclosed subject matter, it is possible to provide a medical apparatus, medical apparatus control method, and medical apparatus cooperation system in which, in the case where medical apparatuses communicate with each other, unintended connection switching can be prevented from occurring.

## Claims

1. A medical apparatus which is communicable with a plurality of CPR assisting devices, the medical apparatus comprising:
a connection destination determining unit which is configured to determine a connection destination CPR assisting device that is a connection destination, from the plurality of CPR assisting devices based on a predetermined rule; and
a communicating unit which is configured to communicate only with the connection destination CPR assisting device.

2. The medical apparatus according to claim 1, wherein
the connection destination determining unit determines the connection destination CPR assisting device from the plurality of CPR assisting devices based on a connection list which defines a connectable device.

3. The medical apparatus according to claim 2, wherein
the connection list defines devices and priority levels while correlating the devices with the priority levels.

4. The medical apparatus according to claim 1, wherein
the connection destination determining unit determines, among the plurality of CPR assisting devices, a CPR assisting device which transmits a connection request at an earliest timing, as the connection destination CPR assisting device.

5. The medical apparatus according to claim 1, wherein
the connection destination determining unit determines the connection destination CPR assisting device from the plurality of CPR assisting devices based on attribute information which is received from each of the plurality of CPR assisting devices.

6. The medical apparatus according to claim 1, wherein
the connection destination determining unit determines the connection destination CPR assisting device from the plurality of CPR assisting devices based on a history list storing CPR assisting devices with which the medical apparatus has communicated.

7. The medical apparatus according to claim 1, further comprising:
an inputting unit which is configured to receive an input from a user, wherein
the connection destination determining unit determines the connection destination CPR assisting device from the plurality of CPR assisting devices in accordance with an input from the inputting unit.

8. The medical apparatus according to any one of claims 1 to 7, wherein
the connection destination CPR assisting device is a single CPR assisting device.

9. A method of controlling a medical apparatus which is communicable with a plurality of CPR assisting devices, the method comprising:
determining a connection destination CPR assisting device that is a connection destination, from the plurality of CPR assisting devices based on a predetermined rule; and
communicating only with the connection destination CPR assisting device.

10. A medical apparatus cooperation system including a plurality of CPR assisting devices and a medical apparatus which is communicable with the plurality of CPR assisting devices, wherein
each of the plurality of CPR assisting devices transmits a connection request to the medical apparatus, and
the medical apparatus includes:
a connection destination determining unit which is configured to determine a connection destination CPR assisting device that is a connection destination, from the plurality of CPR assisting devices based on a predetermined rule; and
a communicating unit which is configured to communicate only with the connection destination CPR assisting device.

11. A CPR assisting device which is configured to transmit a connection request to a medical apparatus that is configured to determine a connection destination from a plurality of devices and that is configured to communicate with the connection destination, and which, only when the CPR assisting device is determined as the communication destination by the medical apparatus, is configured to perform a communication process with the medical apparatus.

12. A medical apparatus which is communicable with a plurality of opposing medical apparatuses, the medical apparatus comprising:
a connection destination determining unit which is configured to determine a connection destination opposing medical apparatus that is a connection destination, from the plurality of opposing medical apparatuses based on a predetermined rule; and
a communicating unit which communicates only with the connection destination opposing medical apparatus.
